(19) Europäisches Patentamt
European Patent Office
Office européen des brevets

(11) Veröffentlichungsnummer: **0 237 489**
B1

(12) # EUROPÄISCHE PATENTSCHRIFT

(45) Veröffentlichungstag der Patentschrift: **29.11.89**

(51) Int. Cl.⁴: **C 07 C 149/24,** C 07 C 149/18

(21) Anmeldenummer: **87810130.2**

(22) Anmeldetag: **06.03.87**

(54) **Umsetzungsprodukte von Bis-glycidylthioethern.**

(30) Priorität: **13.03.86 CH 1020/86**

(43) Veröffentlichungstag der Anmeldung: **16.09.87 Patentblatt 87/38**

(45) Bekanntmachung des Hinweises auf die Patenterteilung: **29.11.89 Patentblatt 89/48**

(84) Benannte Vertragsstaaten: **BE DE FR GB IT**

(56) Entgegenhaltungen:
**EP-A- 0 166 696**
**FR-A- 2 379 516**
**GB-A- 2 172 284**

(73) Patentinhaber: **CIBA-GEIGY AG, Klybeckstrasse 141, CH-4002 Basel (CH)**

(72) Erfinder: **Wirth, Hermann, O., Dr., Lessingstrasse 24, D-6140 Bensheim 3 (DE)**
Erfinder: **Schneider, Rainer, Dr., Am Markstein 4, D-6144 Zwingenberg (DE)**
Erfinder: **Friedrich, Hans-Helmut, Am Rauhenstein 8, D-6147 Lautertal 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 237 489 B1

ACTORUM AG

**Beschreibung**

Die vorliegende Erfindung betrifft neue Umsetzungsprodukte von Bis-glycidylthioethern, deren Verwendung in polaren funktionellen Flüssigkeiten, insbesondere auf Wasserbasis, sowie solche funktionelle Flüssigkeiten enthaltend diese neuen Umsetzungsprodukte.

Sauerstoffhaltige Bis-glycidylthioether sind in der US-A 2 731 437 als flüssige Polymere zur Herstellung von Gelen, Harzen oder Kautschuken beschrieben.

Gegenstand der vorliegenden Erfindung sind Verbindungen der Formel I

$$R^2\text{-}Y\text{-}\left[CH_2\text{-}\underset{\displaystyle OH}{\underset{|}{CH}}\text{-}CH_2\text{-}S\text{-}R^1\text{-}S\text{-}CH_2\text{-}\underset{\displaystyle OH}{\underset{|}{CH}}\text{-}CH_2\text{-}Y\right]_n\text{-}R^2 \quad (I),$$

worin n eine Zahl von 1 bis 100 ist, Y $-N(R^3)-$, -S- oder $-S-R^4-S-$ darstellt, $R^1$ $C_2$-$C_8$-Alkylen ist, das gegebenenfalls durch 1 bis 3 Ether-Sauerstoffatome unterbrochen sein kann, $R^2$ Wasserstoff, unsubstituiertes oder durch 1 bis 5 Hydroxygruppen substituiertes $C_1$-$C_8$-Alkyl, das gegebenenfalls durch -O-, -S- oder $-N(R^5)-$ einfach oder mehrfach unterbrochen sein kann, bedeutet, mit der Massgabe, dass, wenn Y -S- und n gleich 1 sind, $R^2$ nicht unsubstituiertes $C_1$-$C_8$-Alkyl sein kann, ferner $R^3$ Wasserstoff, unsubstituiertes oder durch 1 bis 5 Hydroxygruppen substituiertes $C_1$-$C_8$-Alkyl darstellt, das gegebenenfalls durch -O-, $-N(R^5)-$ oder -S- einfach oder mehrfach unterbrochen sein kann, $R^4$ unsubstituiertes oder durch -OH einfach oder mehrfach substituiertes $C_2$-$C_8$-Alkylen, das gegebenenfalls durch -O- oder -S- einfach oder mehrfach unterbrochen sein kann, bedeutet und $R^5$ die gleiche Bedeutung wie $R^3$ hat, mit Ausnahme von durch Heteroatome unterbrochenem Alkyl.

Stellt $R^1$ $C_2$-$C_8$-Alkylen dar, so kann dieses Alkylen geradkettig oder verzweigt, bevorzugt aber geradkettig, sein, wie beispielsweise Ethylen, Propylen, Trimethylen, Tetramethylen, Pentamethylen, 2,2-Dimethyl-1,3-trimethylen, Hexamethylen, Heptamethylen oder Octamethylen.

Stellt $R^1$ durch 1 bis 3 Ether-Sauerstoffatome unterbrochenes $C_2$-$C_8$-Alkylen dar, handelt es sich beispielsweise um 2-Oxa-1,3-propylen, 2,4-Di-oxa-1,5-pentylen, 2,5,8-Tri-oxa-1,9-nonylen, 2,5,8-Tri-oxa-1,11-undecylen oder um 2,9-Di-oxa-1,10-decylen.

Stellen $R^2$ und $R^3$ $C_1$-$C_8$-Alkyl dar, so handelt es sich um geradkettiges oder verzweigtes Alkyl, wie beispielsweise um Methyl, Ethyl, n-Propyl, Isopropyl, n-Butyl, Isobutyl, sec.-Butyl, tert.-Butyl, geradkettiges oder verzweigtes Pentyl, Hexyl, Heptyl oder um Octyl.

Stellen $R^2$ und $R^3$ durch 1 bis 5 Hydroxygruppen substituiertes $C_1$-$C_8$-Alkyl dar, so trägt das C-Atom, das direkt an Y gebunden ist, vorzugsweise keine OH-Gruppe.

Stellt $R^2$ bzw. $R^3$ $C_1$-$C_8$-Alkyl dar, das durch -O-, -S- oder $-N(R^5)-$ einfach oder mehrfach unterbrochen ist, so können sich die Heteroatome in jeder möglichen Position befinden.

Stellt $R^4$ $C_2$-$C_8$-Alkylen dar, so kann dieses Alkylen geradkettig oder verzweigt, bevorzugt aber geradkettig, sein, wie beispielsweise Ethylen, Propylen, Trimethylen, Tetramethylen, 2,2-Dimethyl-1,3-trimethylen, Hexamethylen, Heptamethylen oder Octamethylen.

Stellt $R^4$ durch -OH einfach oder mehrfach substituiertes $C_2$-$C_8$-Alkylen das, so ist die Substitution in jeder Position möglich.

Stellt $R^4$ durch -O- oder -S- einfach oder mehrfach unterbrochenes $C_2$-$C_8$-Alkylen dar, handelt es sich beispielsweise um 2-Oxa-1,3-propylen, 2,4-Di-oxa-1,5-pentylen, 3,5-Di-oxa-1,6-hexylen, 3-Oxa-1,6-hexylen, 3,6-Di-oxa-1,8-octylen, 2,5,8-Tri-oxa-1,9-nonylen, 2,5,8-Tri-oxa-1,11-undecylen, 2,9-Di-oxa-1,10-dedylen, 2-Thia-1,3-propylen, 2,4-Di-thia-1,5-pentylen, 3-Thia-1,6-hexylen oder 2,5,8-Tri-thia-1,11-undecylen.

Bevorzugt sind Verbindungen der Formel I, worin $n > 1$ ist.

Eine weitere Ausführungsform betrifft Verbindungen der Formel I, worin Y $-N(R^3)-$ bedeutet.

Von speziellen Interesse sind Verbindungen der Formel I, worin $R^3$ durch 1 bis 5 Hydroxygruppen substituiertes $C_1$-$C_8$-Alkyl darstellt, das gegebenenfalls durch $-N(R^5)-$ einfach oder mehrfach unterbrochen ist.

Insbesondere interessant sind Verbindungen der Formel I, worin $R^3$ durch 5 Hydroxygruppen substituiertes $C_6$-Alkyl bedeutet.

Ganz besonders interessant sind Verbindungen der Formel I, worin Y $-N(R^3)-$ oder -S-, $R^1$ $C_2$-$C_6$-Alkylen, insbesondere Ethylen, $R^2$ Wasserstoff, unsubstituiertes oder mit 1-5 Hydroxygruppen substituiertes $C_1$-$C_6$-Alkyl, das gegebenenfalls durch 1-3 -O- oder -S-Gruppen unterbrochen ist, und $R^3$ Wasserstoff oder mit 1-5 Hydroxygruppen substituiertes $C_1$-$C_8$-Alkyl bedeuten, mit der Massgabe, dass, wenn Y -S- und n gleich 1 sind, $R^2$ nicht unsubstituiertes $C_1$-$C_6$-Alkyl sein kann.

Der Polymerisationsgrad n lässt sich bekanntlich über die Viskosität, zumindest semiquantitativ, ausdrücken in dem Sinne je höher das Molekulargewicht desto höher die Viskosität.

Besonders bevorzugt sind Verbindungen der Formel I, vor allem solche, worin Y $-N(R^3)-$ mit $R^3$ als durch 5 Hydroxygruppen substituiertem $C_6$-Alkyl ist, und n einen solchen Wert besitzt, dass die kinematische Viskosität einer 50%igen Lösung in Wasser bei 40°C 20-50 $mm^2/s$ beträgt.

Ganz speziell bevorzugt sind Verbindungen der Formel I, worin n einen solchen Wert besitzt, dass die kinematische Viskosität einer 50%igen Lösung in Wasser bei 40°C 25-35 $mm^2/s$ beträgt.

Insbesondere bevorzugt sind Verbindungen der Formel I, worin n einen solchen Wert besitzt, dass die kinematische Viskosität einer 50%igen Lösung in Wasser bei 40°C 27-30 $mm^2/s$ beträgt.

Beispiele für Verbindungen der Formel I sind:

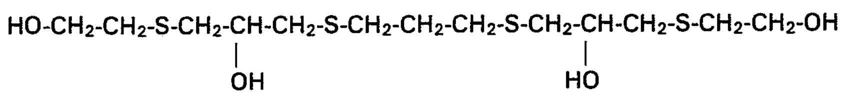

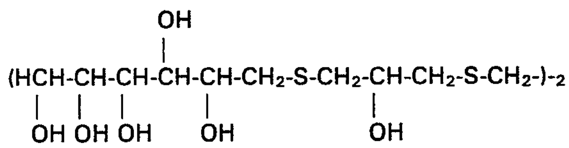

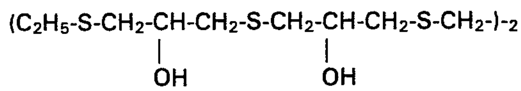

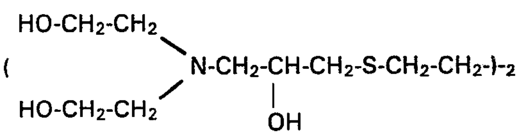

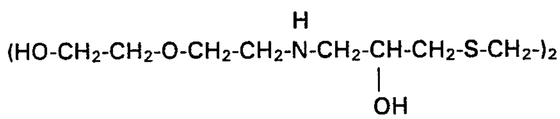

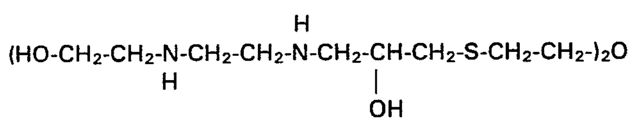

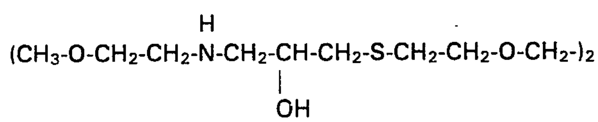

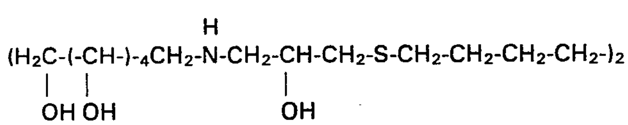

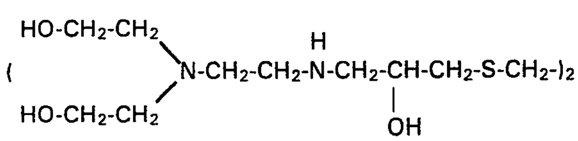

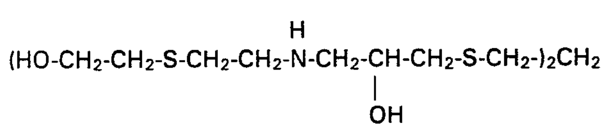

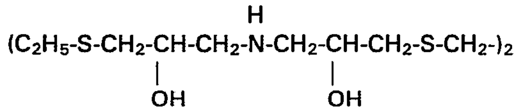

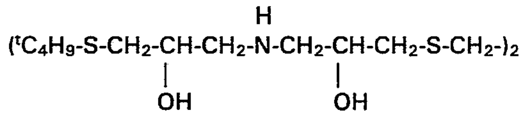

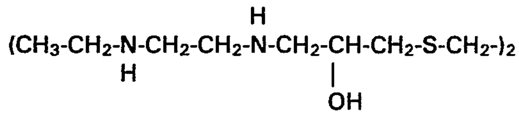

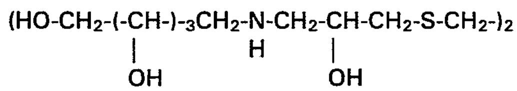

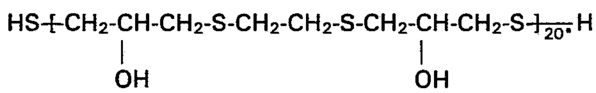

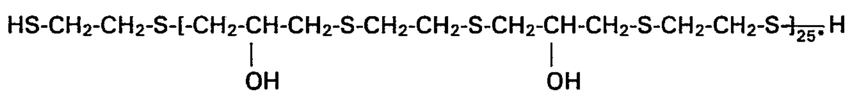

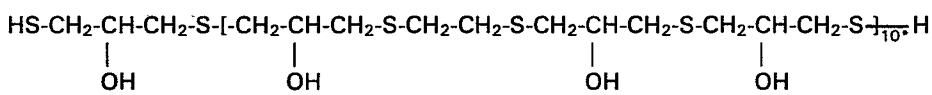

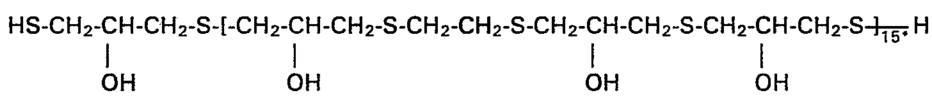

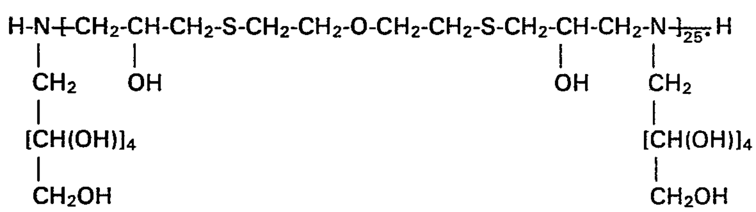

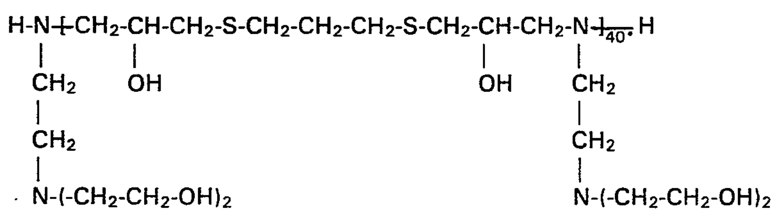

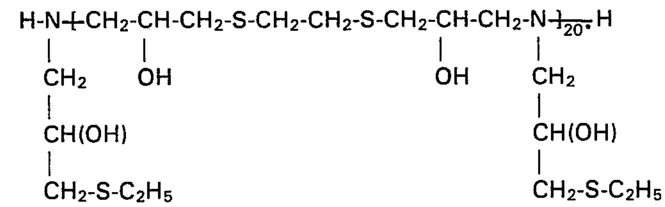

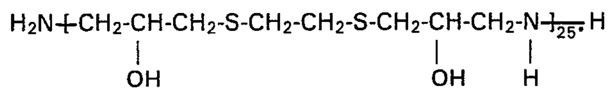

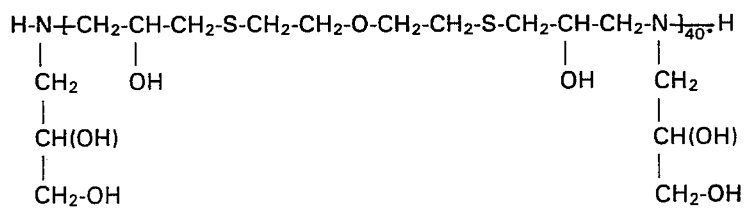

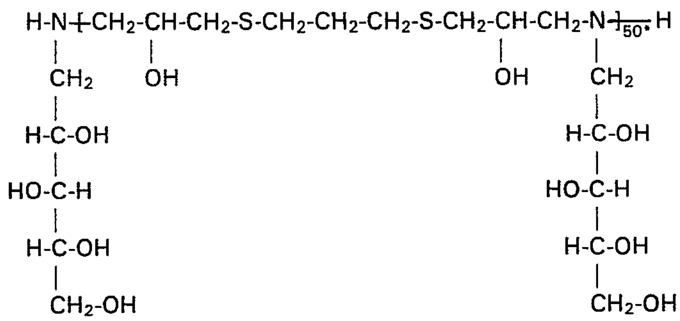

* Diese Zahlen stellen statistische Mittelwerte dar, d.h. die Produkte bestehen aus einem polymolekularen Gemisch, wobei die angegebene Zahl einen Mittelwert bedeutet.

Die Verbindungen der Formel I können z.B. durch Umsetzung von entsprechenden Bis-glycidylthioethern, deren Herstellung z.B. in der EP-A 166 695 beschrieben ist, mit Mercaptanen oder Aminen in an sich bekannter Weise hergestellt werden.

Die Erfindung betrifft auch Reaktionsprodukte, erhältlich aus der Umsetzung von Bis-glycidylthioethern der Formel

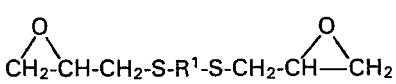

mit Verbindungen der Formel $H\text{-}Y\text{-}R^2$, worin Y $-N(R^3)-$, -S- oder $-S-R^4-S-$ darstellt, $R^1$ $C_2$-$C_8$-Alkylen ist, das gegebenenfalls durch 1 bis 3 Ether-Sauerstoffatome unterbrochen sein kann, $R^2$ Wasserstoff, unsubstituiertes oder durch 1 bis 5 Hydroxygruppen substituiertes $C_1$-$C_8$-Alkyl, das gegebenenfalls durch -O-, -S- oder $-N(R^5)-$ einfach oder mehrfach unterbrochen sein kann, bedeutet, $R^3$ Wasserstoff, unsubstituiertes oder durch 1 bis 5 Hydroxygruppen substituiertes $C_1$-$C_8$-Alkyl darstellt, das gegebenenfalls durch -O-, $-N(R^5)-$ oder -S- einfach oder mehrfach unterbrochen sein kann, $R^4$ unsubstituiertes oder durch -OH einfach oder mehrfach substituiertes $C_2$-$C_8$-Alkylen, das gegebenenfalls durch -O- oder -S- einfach oder mehrfach unterbrochen sein kann, bedeutet und $R^5$ die gleiche Bedeutung wie $R^3$ hat, mit Ausnahme von durch Heteroatome unterbrochenem Alkyl, wobei die Reaktion so geführt wird, dass im Fall Y = S und $R^2$ = unsubstituiertes $C_1$-$C_8$-Alkyl im Reaktionsprodukt mindestens 2 Bis-glycidylthioethereinheiten pro Molekül enthalten sind, sowie ferner ein Verfahren zur Herstellung von Verbindungen der Formel I bzw. der vorstehend beschriebenen Reaktionsprodukte, das dadurch gekennzeichnet ist, dass man einen Bis-glycidylthioether der Formel

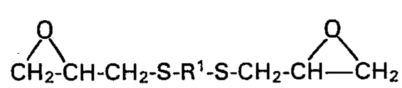

mit einer Verbindung der Formel $H\text{-}Y\text{-}R^2$ umsetzt, wobei die allgemeinen Symbole wie vorstehend definiert sind.

Die Gleichungen (1) und (2) erläutern die Additionsreaktionen für je ein allgemeines Beispiel.

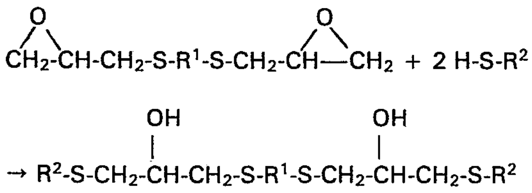

(1)

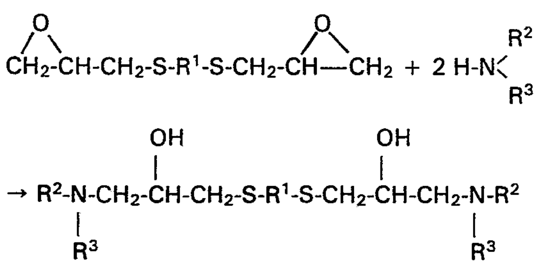

(2)

Als Reaktionsmedien werden bevorzugt Wasser, Methanol oder Ethylenglycole bzw. Polyethylenglycole oder Mischungen der drei letztgenannten mit Wasser verwendet.

Vorteilhaft wird dabei so verfahren, dass eine möglichst hohe, für die Konfektionierung geeignete Konzentration des Endproduktes resultiert. Zweckmässig sind die Reaktionsmedien identisch mit den funktionellen Flüssigkeiten bzw. sie sind Bestandteile davon, in denen die Endprodukte eingesetzt werden können (siehe unten).

In gewissen Fällen können die Reaktionspartner per se ohne Verwendung eines Reaktionsmediums umgesetzt werden.

Die Additionsreaktion mit Mercaptanen ist zweckmässig mit anorganischen Basen (z.B. Kaliumhydroxid) oder auch tertiären Aminen zu katalysieren. Sie verläuft bereits bei Raumtemperatur; durch Arbeiten bei höheren Temperaturen, z.B. bei 50-70°C, lassen sich aber die Reaktionszeiten wesentlich verkürzen.

Die Additionsreaktion mit Aminen erfordert im allgemeinen keine Katalyse. Um eine ausreichende Reaktionsgeschwindigkeit zu gewährleisten, ist es zweckmässig, bei erhöhter Temperatur, z.B. bei 50-80°C, zu arbeiten.

Verbindungen der Formel I, worin n einen Wert > 1 hat, werden analog hergestellt. Es sei noch einmal hervorgehoben, dass n einen statistischen Mittelwert darstellt, da bei diesen Polyadditionen Produkte entstehen, die durch eine Molekulargewichtsverteilung gekennzeichnet sind. In diesem Fall wird ein Bis-glycidylthioether mit einem Bis-mercaptan der Formel $H\text{-}S\text{-}R^4\text{-}S\text{-}H$ oder $H_2S$ oder einem sterisch nicht gehinderten primären Amin der Formel $H_2N\text{-}R^3$ umgesetzt, wobei beide Reaktionspartner im molaren Verhältnis von 1:1 eingesetzt werden.

Durch Vorgabe des stöchiometrischen Verhältnisses der beiden bifunktionellen Komponenten hat man es in der Hand, auf n Einfluss zu nehmen. Ein von 1:1 abweichendes molares Verhältnis führt immer zu einem erniedrigten n.

Als Makromoleküle begrenzende Endgruppen kommen die freien SH- oder NH-Gruppen in Frage. Dabei sind die SH- oder NH-Gruppen dominant, wenn das Bis-mercaptan oder das Amin als Edukt überwiegt.

Die erfindungsgemässen Verbindungen der Formel I stellen sehr gute Hochdruck- und Verschleissschutz-Additive für polare funktionelle Flüssigkeiten dar. Ausserdem tragen sie — zumindest mittelbar — zum Korrosionsschutz bei. Schliesslich können die höhermolekularen Vertreter auch noch als Verdicker (Viskositätssteigerer) eingesetzt werden.

Die erfindungsgemässen Verbindungen der Formel I sind von polarer Natur, d.h. es handelt sich um Substanzen mit polarer Löslichkeitscharakteristik. Die Verbindungen der Formel I können in polaren funktionellen Flüssigkeiten, insbesondere auf der Basis von Wasser, Ethylenglycol, Diethylenglycol, Polyethylenglycol und ihren Abmischungen mit Wasser in homogenem Lösungszustand eingesetzt werden. Ebenso können sie auch in funktionellen Flüssigkeiten auf der Basis von Phosphorsäureestern, bevorzugt Phosphorsäure-arylestern eingesetzt werden. Aber auch kolloidale auch kolloidale Systeme sind technisch sehr wertvoll, insbesondere aufgrund ihrer günstigen Dispergier- und Emulgiereigenschaften, z.B. für die Herstellung von Emulsionen und Mikroemulsionen.

Die Verbindungen der Formel I können speziell auch in polaren, komplex aufgebauten funktionellen Flüssigkeiten mit Wasser als vorherrschender Basisflüssigkeit, insbesondere mit einem Wasseranteil von mindestens 50 Gew.-%, und gegebenenfalls mit einem Gehalt an niedermolekularen Glycol-Verbindungen, wie z.B. Ethylenglycol, Diethylenglycol oder Thiodiethylenglycol, ferner mit Phosphorsäure sowie einer Base zur Einstellung des pH-Wertes zwischen 7,5 und 12, vorzugsweise bei 8,5, als weitere Komponenten, eingesetzt werden.

Basen zur pH-Regulierung sind z.B. Alkalihydroxide, vorzugsweise Kaliumhydroxid, wasserlösliche organische Basen, vorzugsweise Alkanolamine, insbesondere Ethanolamin. Auch polymere Amine, wie z.B. Polyethylenimin sind für die pH-Regulierung einsetzbar.

Die vorstehende, komplex aufgebaute funktionelle Flüssigkeit kann als weitere Komponenten noch Fettsäuren, vorzugsweise Ölsäure, und gegebenenfalls auch Borsäure enthalten.

Die polaren, komplex aufgebauten funktionellen Flüssigkeiten können noch zusätzliche Additive enthalten, die zugegeben werden, um die Grundeigenschaften von diesen funktionellen Flüssigkeiten noch weiter zu verbessern; dazu gehören: Korrosionsschutzmittel, Schaumbrecher, Enthärter und Biocide.

Beispiele für Korrosionsinhibitoren sind folgende:

a) Organische Säuren, ihre Ester, Ammonium-, Amin-, Alkanolamin- und Metallsalze wie z.B. Benzoesäure, p-tert.-Butyl-benzoesäure, Di-Na-sebacat, Triethanolaminlaurat, Isononansäure, Triethanolaminsalz der p-Toluol-sulfonamidocapronsäure, Triethanolaminsalz der Benzol-sulfonamidocapronsäure, Triethanolaminsalze der 5-Ketocarbonsäureabkömmlinge, wie sie in der EP-A 41 927 beschrieben sind, Natrium-N-Lauroyl-sarcosinat oder Nonylphenoxyessigsäure.

b) Stickstoffhaltige Stoffe wie z.B. Fettsäurealkanolamide, Imidazoline wie z.B. 1-Hydroxy-ethyl-2--oleyl-imidazolin, Oxazoline, Triazole wie z.B. Benztriazole oder deren Mannich-Basen-Derivate, Triethanolamine, Fettamine, anorganische Salze wie z.B. Natriumnitrat, und die in der EP-A- 46 139 beschriebenen Carboxy-Triazinverbindungen.

c) Phosphorhaltige Stoffe wie z.B. Aminphosphate, Phosphonsäuren oder anorganische Salze wie z.B. Na-Dihydrogenphosphat oder Zinkphosphat.

d) Schwefehaltige Verbindungen wie z.B. Natrium-, Calcium- oder Barium-Petrolsulfonate oder heterocyclische Verbindungen wie z.B. Na-Mercaptobenzthiazol.

Komplexierungsmittel, wie Nitrilotriessigsäure und deren Salze, Schaum-brechende Mittel, wie Silikone wie z.B. Polydimethylsiloxane, Distearylsebacamid, Distearyladipamid und ähnliche von Ethylenoxid- und/oder Propylenoxidkondensationen abgeleitet Produkte, Additionsprodukte von Fettalkoholen, wie Caprylalkohole und deren Kondensationsprodukte mit Ethylenoxid, ferner Biocide wie z.B. Amine, quaternäre Ammoniumverbindungen, Chlorphenole, schwefelhaltige Verbindungen, wie Sulfone, Methylen-bis-thiocyanate und -Carbamate, Isothiazolone, Brompropionamide, Triazine, Phosphoniumverbindungen, Chlor und Chlor abgebende Stoffe und Organometallverbindungen wie Tributyl-zinnoxid können zusätzlich eingesetzt werden.

Ein besonderer Vorteil der erfindungsgemässen Verbindungen ist ihre hydrolytische Stabilität. Sie zeigen daher eine äusserst geringe pH-Sensibilität.

Auf die viskositätssteigernde Wirkung der Polyaddukte sei nochmals besonders hingewiesen; auch dies ist ein sehr günstiger Aspekt, da polare funktionelle Flüssigkeiten mit sehr hohem Wassergehalt die Anwendung eines viskositätssteigernden Additivs (Verdickers) erforderlich machen.

Für den Einsatz von Verbindungen der Formel I in Hydraulikflüssigkeiten ist die Mitverwendung eines zusätzlichen Verdickers als weitere komponente vorteilhaft.

Als Verdicker kommen in Frage z.B. Polyalkylenoxide, Polyalkylmethacrylate, Polyamidester, Polyamid-alkoxylate oder Polyethylenimine, die gleichzeitig vollständig oder partiell die Funktion eines pH-Regulators übernehmen können.

Die Einsatzmenge der Verdicker liegt zweckmässig zwischen 2 und 50 Gew.-%, vorzugsweise zwischen 5 und 15 Gew.-%, bezogen auf die Basisflüssigkeit.

Die Einsatzmenge der Verbindungen der Formel I beträgt beispielsweise 0,5 bis 10 Gew.-%, bezogen auf die Gesamtmenge der polaren funktionellen Flüssigkeit.

Werden die Verbindungen der Formel I in komplex aufgebauten funktionellen Flüssigkeiten eingesetzt, so beträgt ihre Einsatzmenge zweckmässig 0,5-2 Gew.-%, vorzugsweise jedoch 0,5-1 Gew.-%, bezogen auf die Gesamtmenge der komplex aufgebauten funktionellen Flüssigkeit, wobei die Gesamtmenge der zugesetzten Additive vorzugsweise 0,5-10 Gew.-%, vorzugsweise 2,5-5 Gew.-%, bezogen auf deren Wasseranteil, ausmacht.

Polare funktionelle Flüssigkeiten, insbesondere solche auf der Basis von Wasser, Ethylenglykol, Diethylenglycol, Polyethylenglycol und deren Abmischungen mit Wasser oder auf der Basis von Phosphorsäureestern sowie komplex aufgebaute funktionelle Flüssigkeiten können beispielsweise als Hydraulikflüssigkeiten, Metallbearbeitungsflüssigkeiten, Kühlflüssigkeiten oder als «Drilling-fluids», insbesondere als Hydraulikflüssigkeiten, eingesetzt werden.

Die vorliegende Erfindung betrifft auch eine Zusammensetzung enthaltend eine polare funktionelle Flüssigkeit, insbesondere auf der Basis von Wasser, Ethylenglycol, Diethylenglycol, Polyethylenglycol und deren Abmischungen mit Wasser sowie auf der Basis von Phosphorsäureestern oder eine komplex aufgebaute funktionelle Flüssigkeit, und mindestens eine Verbindung der Formel I.

Die nachfolgenden Beispiele erläutern die Erfindung weiter. Prozent und Teile beziehen sich darin, ebenso wie in der übrigen Beschreibung und in den Patentansprüchen, auf das Gewicht, wenn nichts anderes angegeben ist.

*Beispiel 1:*

Additionsprodukt von Glucamin an 1,2-Bis(glycidylmercapto)-ethan

*Stufe 1:* 1,2-Bis(glycidylmercapto)-ethan

In einem 1 l SOVIREL® -Reaktor (doppelwandiges Reaktionsgefäss) mit Thermometer, Rührer, Tropftrichter, Dosiertropftrichter und pH-Elektrode werden 141 g 1,2-Dimercaptoethan und 4 g Tetrabutylammoniumchlorid vorgelegt. Zu dieser Lösung werden unter Wasserkühlung und Rühren 278 g Epichlorhydrin innerhalb 200 Min. bei einem pH-Wert von 9,9-10,1 eingetropft. Der pH-Wert wird durch Dosierung von 20%iger wässriger Natriumhydroxidlösung über den gesamten Zeitraum von 200 Min. konstant gehalten (Verbrauch ca. 8 ml). Die Reaktionstemperatur sollte 20°C nicht übersteigen. Nach beendeter exothermer Reaktion werden 530 ml 20%ige Natriumhydroxidlösung innerhalb 15 Min. eingetropft, das Reaktionsgemisch 20 Min. bei 40°C nachgerührt und schliesslich die wässrige Phase scharf abgetrennt. Die verbleibende organische Phase ist das Bis-epoxid.

Die Ausbeute beträgt 293 g (95% d.Th.) mit einem Brechungsindex von $n_D^{20}$: 1,5430 und einem Epoxid-Gehalt von 96,5 Mol-%.

*Stufe 2:*

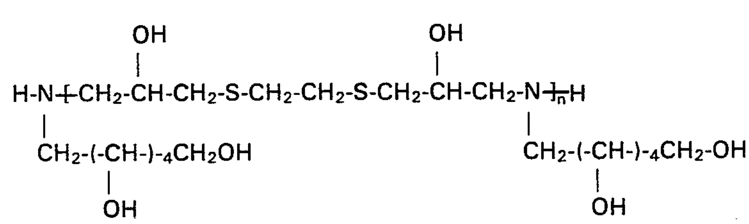

32,2 g Glucamin werden in 77,4 g dest. Wasser gelöst. Zu dieser Lösung werden unter Rühren bei 60-70°C innerhalb 45 Min. 41,2 g 1,2-Bis(glycidylmercapto)-ethan eingetropft; stark exotherme Reaktion. Das Gemisch wird 30 Min. bei gleicher Temperatur nachgerührt.

Ausbeute: 154,8 g ≙ 100% d.Th. einer gelblichen viskosen Flüssigkeit mit einem Brechungsindex einer 50%igen Lösung in Wasser von $n_D^{20}$: 1,4455 und einer Viskosität bei 40°C von 27-30 $mm^2/s$ (ebenfalls 50%ige Lösung in Wasser).

*Beispiel 2:*

OH<br>|<br>$(HO-CH_2-CH_2-S-CH_2-CH-CH_2-S-CH_2)_2$

14,1 g 2-Mercaptoethanol werden mit katalytischen Mengen Natriummethylat versetzt, unter Rühren auf 60-70°C erwärmt und nach dem Entfernen des Heizbades 20,6 g 1,2-Bis(glycidylmercapto)-ethan bei der gleichen Temperatur eingetropft (starke exotherme Reaktion). Nach beendeter Zugabe wird das Gemisch nach 30 Minuten bei 70°C nachgerührt.

Ausbeute: 34,2 g ≙ 100% d.Th., kristalline Masse $n_D^{50}$: 1,5840

*Beispiele 3-10:*

Analog zu Beispiel 1, 2. Stufe werden die Verbindungen der Beispiele 3-9 und analog zu Beispiel 2 wird die Verbindung des Beispiels 10 hergestellt.

| Beispiel | Formel | Physikal. Daten |
|---|---|---|
| 3 | OH<br>\|<br>$(HO-CH_2-CH_2-NH-CH_2-CH-CH_2-S-CH_2)_2$ | 50%ig in $H_2O$<br>$n_D^{20}$: 1,4390 |
| 4 | $HO-CH_2$ OH<br>\| \|<br>$(HO-CH_2-C-NH-CH_2-CH-CH_2-S-CH_2)_2$<br>\|<br>$HO-CH_2$ | 50%ig in $H_2O$<br>$n_D^{20}$: 1,4372 |
| 5 | OH $CH_3$ OH<br>\| \| \|<br>$(CH_2-CH-CH-CH-CH-CH_2-N-CH_2-CH-CH_2-S-CH_2)_2$<br>\| \| \| \|<br>OH OH OH OH | 50%ig in $H_2O$<br>$n_D^{20}$: 1,4376 |
| 6 | OH $C_2H_5$ OH<br>\| \| \|<br>$(CH_2-CH-CH-CH-CH-CH_2-N-CH_2-CH-CH_2-S-CH_2)_2$<br>\| \| \| \|<br>OH OH OH OH | 50%ig in $H_2O$<br>$n_D^{20}$: 1,436 |
| 7 | 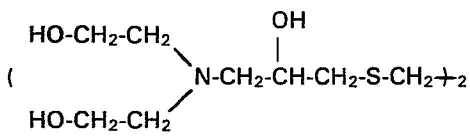 | $n_D^{20}$: 1,542 |
| 8 | OH OH<br>\| \|<br>$H-N-(CH_2-CH-CH_2-S-CH_2-CH_2-S-CH_2-CH-CH_2-N)_n-H$<br>\| \|<br>$(CH_2)_3$ $(CH_2)_3$<br>\| \|<br>$(N-(-CH_2-CH_2-OH)_2$ $N-(-CH_2-CH_2-OH)_2$ | 50%ig in $H_2O$<br>$n_D^{20}$: 1,4447 |

| Beispiel | Formel | Physikal. Daten |
|---|---|---|
| 9 | OH OH<br>│ │<br>$H-N\{-CH_2-CH-CH_2-S-CH_2-CH_2-S-CH_2-CH-CH_2-N-\}_n H$<br>│ │<br>$CH_2-CH_2-OH$ $CH_2-CH_2-OH$ | $n_D^{50}$: 1,5755 |
| 10 | OH<br>│<br>$CH_2-S-CH_2-CH_2-S-CH_2-CH-CH_2-]-S-(-CH_2-CH_2-O-)-_2CH_2-CH_2-SH$<br>│<br>HO-CH<br>│<br>$CH_2-S-(-CH_2-CH_2-O-)-_2CH_2-CH_2-S-]_n H$ | $n_D^{50}$: 1,5768 |

*Beispiel 11:*

Mit dem Shell-Vierkugel-Apparat (IP 239/73 Extreme pressure and wear lubricant test for oils and greases — four ball machine) werden folgende Werte bestimmt:

1. W.L. = Weld load [Schweisslast in Newton (N)]. Das ist die Last, bei der die 4 Kugeln innerhalb von 10 Sekunden zusammenschweissen.
2. W.S.D. = Wear Scar Diameter in mm: Das ist der mittlere Verschleissdurchmesser bei einer Belastung von 400 N während 10 Minuten.

Als Testflüssigkeit für die Wirksamkeit wird deionisiertes Wasser verwendet.

| Additiv gemäss Beispiel Nr. | W.L. (N) 2,5% Additiv | W.S.D. (mm) 2,5% Additiv |
|---|---|---|
| 1 | 2200 | 0.65 |
| 2 | 2000 | 0.65 |
| 3 | 2000 | 0.76 |
| 4 | 1800 | 0.75 |
| 5 | <1600 | 0.77 |
| 6 | 1400 | 0.75 |
| 7 | <1400 | 0.60 |
| 8 | 2000 | 0.73 |

*Beispiele 12-14:*

Folgende komplex aufgebaute funktionelle Flüssigkeiten werden hergestellt:

TABELLE 1

| Zusammensetzung | Bsp. 12 | Bsp. 13 | Bsp. 14[1)] |
|---|---|---|---|
| Wasser | 97,50% | 97,25% | 94,50% |
| Phosphorsäure | 0,63% | 0,80% | 0,70% |
| Borsäure | 0,06% | — | — |
| Äthanolamin | 0,87% | — | 0,88% |
| KOH | — | 0,87% | — |
| Ölsäure | 0,16% | 0,18% | 0,15% |
| Verbindung n. Beispiel 1, Stufe 2 | 0,87% | 0,90% | 0,77% |
| Polyalkylenglycol (Verdicker) | — | — | 3,00% |

1) Viskosität bei 40°C: 20,9 cSt.

*Beispiel 15:*

Analog zu Beispiel 11 werden die Weld Load in N und der Wear Scar Diameter in mm mit dem Shell-Vierkugelapparat bestimmt, wobei als Testflüssigkeiten die funktionellen Flüssigkeiten der Beispiele 12 bis 14 verwendet werden.

TABELLE 2

| Zusammensetzung gemäss Bsp. Nr. | WL (N) | WSD (mm) |
|---|---|---|
| 12 | 2400 | 0,50 |
| 13 | 4000 | 0,50 |
| 14 | 2800 | 0,63 |

**Patentansprüche**

1. Verbindungen der Formel I

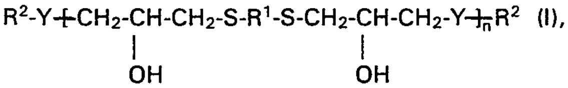

worin n eine Zahl von 1 bis 100 ist, Y -N($R^3$)-, -S- oder -S-$R^4$-S- darstellt, $R^1$ $C_2$-$C_8$-Alkylen ist, das gegebenenfalls durch 1 bis 3 Ether-Sauerstoffatome unterbrochen sein kann, $R^2$ Wasserstoff, unsubstituiertes oder durch 1 bis 5 Hydroxygruppen substituiertes $C_1$-$C_8$-Alkyl, das gegebenenfalls durch -O-, -S- oder -N($R^5$)- einfach oder mehrfach unterbrochen sein kann, bedeutet, mit der Massgabe, dass, wenn Y -S- und n gleich 1 sind, $R^2$ nicht unsubstituiertes $C_1$-$C_8$-Alkyl sein kann, ferner $R^3$ Wasserstoff, unsubstituiertes oder durch 1 bis 5 Hydroxygruppen substituiertes $C_1$-$C_8$-Alkyl darstellt, das gegebenenfalls durch -O-, -N($R^5$)- oder -S- einfach oder mehrfach unterbrochen sein kann, $R^4$ unsubstituiertes oder durch -OH einfach oder mehrfach substituiertes $C_2$-$C_8$-Alkylen, das gegebenenfalls durch -O- oder -S-einfach oder mehrfach unterbrochen sein kann, bedeutet und $R^5$ die gleiche Bedeutung wie $R^3$ hat, mit Ausnahme von durch Heteroatome unterbrochenem Alkyl.

2. Verbindungen gemäss Anspruch 1, worin in Formel I n > 1 ist.

3. Verbindungen gemäss Anspruch 1, worin in Formel I Y -N($R^3$)- bedeutet.

4. Verbindungen gemäss Anspruch 3, worin in Formel I $R^3$ durch 1 bis 5 Hydroxygruppen substituiertes $C_1$-$C_8$-Alkyl darstellt, das gegebenenfalls durch -N($R^5$)- einfach oder mehrfach unterbrochen ist.

5. Verbindungen gemäss Anspruch 4, worin in Formel I $R^3$ durch 5 Hydroxygruppen substituiertes $C_6$-Alkyl bedeutet.

6. Verbindungen gemäss Anspruch 1, worin in Formel I Y -N($R^3$)- oder -S-, $R^1$ Ethylen, $R^2$ Wasserstoff, unsubstituiertes oder mit 1 bis 5 Hydroxygruppen substituiertes $C_1$-$C_6$-Alkyl, das gegebenenfalls durch 1 bis 3 -O- oder -S-Gruppen unterbrochen ist, und $R^3$ Wasserstoff oder mit 1 bis 5 Hydroxygruppen substituiertes $C_1$-$C_8$-Alkyl bedeuten mit der Massgabe, dass, wenn Y -S- und n gleich 1 ist, $R^2$ nicht unsubstituiertes $C_1$-$C_6$-Alkyl sein kann.

7. Verbindungen gemäss einem der Ansprüche 1-5, worin in Formel I n einen solchen Wert besitzt, dass die kinematische Viskosität einer 50%igen Lösung in Wasser bei 40°C 20-50 $mm^2$/S beträgt.

8. Verbindungen gemäss einem der Ansprüche 1-5, worin in Formel I n einen solchen Wert besitzt, dass die kinematische Viskosität einer 50%igen Lösung in Wasser bei 40°C 25-35 $mm^2$/s beträgt.

9. Verbindung gemäss einem der Ansprüche 1-5, worin in Formel I n einen solchen Wert besitzt, dass die kinematische Viskosität einer 50%igen Lösung in Wasser bei 40°C 27-30 $mm^2$/s beträgt.

10. Verwendung der Verbindungen der Formel I gemäss Anspruch 1 als Hochdruck- und Verschleissschutz-Additive in polaren funktionellen Flüssigkeiten.

11. Zusammensetzung enthaltend eine polare funktionelle Flüssigkeit und mindestens eine Verbindung der Formel I gemäss Anspruch 1.

12. Zusammensetzung gemäss Anspruch 11, worin die polare funktionelle Flüssigkeit auf der Basis von Wasser, Ethylenglycol, Diethylenglycol, Polyethylenglycol und deren Abmischungen mit Wasser oder auf der Basis von Phosphorsäureestern oder eine komplex aufgebaute funktionelle Flüssigkeit ist.

13. Zusammensetzung gemäss Anspruch 12, worin in der komplex aufgebauten funktionellen Flüssigkeit der Wasseranteil mindestens 50 Gew.-% beträgt, und worin als weitere Komponenten Phosphorsäure und eine Base zur Einstellung des pH-Wertes zwischen 7,5 und 12 vorhanden sind.

14. Zusammensetzung gemäss Anspruch 13, worin die komplex aufgebaute funktionelle Flüssigkeit zusätzlich Ölsäure und Borsäure enthält.

15. Zusammensetzung gemäss Anspruch 11, worin die Einsatzmenge der Verbindungen der Formel I 0,5-10 Gew.-% beträgt.

16. Reaktionsprodukte, erhältlich aus der Umsetzung von Bis-glycidylthioethern der Formel

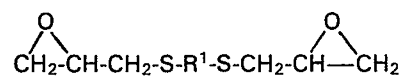

mit Verbindungen der Formel H-Y-$R^2$, worin Y -N($R^3$)-, -S- oder -S-$R^4$-S- darstellt, $R^1$ $C_2$-$C_8$-Alkylen ist, das gegebenenfalls durch 1 bis 3 Ether-Sauerstoffatome unterbrochen sein kann, $R^2$ Wasserstoff, unsubstituiertes oder durch 1 bis 5 Hydroxygruppen substituiertes $C_1$-$C_8$-Alkyl, das gegebenenfalls durch -O-, -S- oder -N($R^5$)- einfach oder mehrfach unterbrochen sein kann, bedeutet, $R^3$ Wasserstoff, unsubstituiertes oder durch 1 bis 5 Hydroxygruppen substituiertes $C_1$-$C_8$-Alkyl darstellt, das gegebenenfalls durch -O-, -N($R^5$)- oder -S- einfach oder mehrfach unterbrochen sein kann, $R^4$ unsubstituiertes oder durch -OH einfach oder mehrfach substituiertes $C_2$-$C_8$-Alkylen, das gegebenenfalls durch -O- oder -S- einfach oder mehrfach unterbrochen sein kann, bedeutet und $R^5$ die gleiche Bedeutung wie $R^3$ hat, mit Ausnahme von durch Heteroatome unterbrochenem Alkyl, wobei die Reaktion so geführt wird, dass im Fall Y = S und $R^2$ = unsubstituiertes $C_1$-$C_8$-Alkyl im Reaktionsprodukt mindestens 2 Bis-glycidylthioethereinheiten pro Molekül enthalten sind.

**Claims**

1. A compound of the formula I

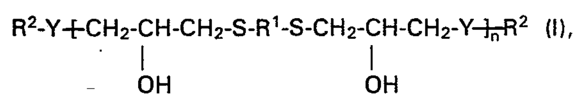

in which n is a number from 1 to 100, Y is -N($R^3$)-, -S- or -S-$R^4$-S-, $R^1$ is $C_2$-$C_8$alkylene, which can be interrupted by 1 to 3 ether oxygen atoms, $R^2$ is hydrogen, unsubstituted or hydroxyl-monosubstituted to -pentasubstituted $C_1$-$C_8$alkyl, which can be inter-

rupted one or more times by -O-, -S- or -N($R^5$)- with the proviso that $R^2$ cannot be unsubstituted $C_1$-$C_8$alkyl when Y is -S- and n is 1, $R^3$ is hydrogen, unsubstituted or hydroxyl-monosubstituted to -pentasubstituted $C_1$-$C_8$alkyl, which can be interrupted one or more times by -O-, -N($R^5$)- or -S-, $R^4$ is unsubstituted or -OH-monosubstituted or -polysubstituted $C_2$-$C_8$alkylene, which can be interrupted one or more times by -O- or -S-, and $R^5$ is defined in the same way as $R^3$ except that it is not alkyl interrupted by heteroatoms.

2. A compound according to claim 1, where, in the formula I, n is > 1.

3. A compound according to claim 1, where, in the formula I, Y is -N($R^3$)-.

4. A compound according to claim 3, where, in the formula I, $R^3$ is hydroxyl-monosubstituted to -pentasubstituted $C_1$-$C_8$alkyl which can be interrupted one or more times by -N($R^5$)-.

5. A compound according to claim 4, where, in the formula I, $R^3$ is hydroxyl-pentasubstituted $C_6$alkyl.

6. A compound according to claim 1, where, in the formula I, Y is -N($R^3$)- or -S-, $R^1$ is ethylene, $R^2$ is hydrogen, unsubstituted or hydroxylmonosubstituted to -pentasubstituted $C_1$-$C_6$alkyl which can be interrupted by 1 to 3 -O- or -S- groups, and $R^3$ is hydrogen or hydroxylmonosubstituted to -pentasubstituted $C_1$-$C_8$alkyl, with the proviso that $R^2$ cannot be unsubstituted $C_1$-$C_6$alkyl when Y is -S- and n is 1.

7. A compound according to any one of claims 1-5, where, in the formula I, n has a value such that the kinematic viscosity of a 50% solution in water at 40°C is 20-50 mm²/s.

8. A compound according to any one of claims 1-5, where, in the formula I, n has a value such that the kinematic viscosity of a 50% solution in water at 40°C is 25-35 mm²/s.

9. A compound according to any one of claims 1-5, where, in the formula I, n has a value such that the kinematic viscosity of a 50% solution in water at 40°C is 27-30 mm²/s.

10. Use of a compound of the formula I according to claim I as a high pressure and antiwear additive in polar functional fluids.

11. A composition containing a polar functional fluid and at least one compound of the formula I according to claim 1.

12. A composition according to claim 11, wherein the polar functional fluids is a functional fluid based on water, ethylene glycol, diethylene glycol, polyethylene glycol and blends thereof with water or on phosphoric acid esters or has a complex structure.

13. A composition according to claim 12, wherein, in the functional fluid of complex structure, the water content is at least 50% by weight and wherein phosphoric acid and a base for setting the pH between 7.5 and 12 are present as further components.

14. A composition according to claim 13, wherein the functional fluid of complex structure additionally contains oleic acid and boric acid.

15. A composition according to claim 11, wherein the amount used of compound of the formula I is 0.5-10% by weight.

16. A reaction product obtainable by reacting a bisglycidyl thioether of the formula

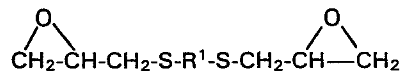

with a compound of the formula H-Y-$R^2$ in which Y is -N($R^3$)-, -S- or -S-$R^4$-S-, $R^1$ is $C_2$-$C_8$alkylene, which can be interrupted by 1 to 3 ether oxygen atoms, $R^2$ is hydrogen, unsubstituted or hydroxyl-monosubstituted to -pentasubstituted $C_1$-$C_8$alkyl, which can be interrupted one or more times by -O-, -S- or -N($R^5$)-, $R^3$ is hydrogen, unsubstituted or hydroxylmonosubstituted to -pentasubstituted $C_1$-$C_8$alkyl, which can be interrupted one or more times by -O-, -N($R^5$)- or -S-, $R^4$ is unsubstituted or -OH- monosubstituted or -polysubstituted $C_2$-$C_8$alkylene, which can be interrupted one or more times by -O- or -S-, and $R^5$ is defined in the same way as $R^3$ except it is not alkyl interrupted by heteroatoms, the reaction being carried out in such a way that in the case of Y = S and $R^2$ = unsubstituted $C_1$-$C_8$alkyl the reaction product contains at least 2 bisglycidyl thioether units per molecule.

**Revendications**

1. Composés répondant à la formule I

$$R^2\text{-}Y\!\left[CH_2\text{-}\underset{OH}{CH}\text{-}CH_2\text{-}S\text{-}R^1\text{-}S\text{-}CH_2\text{-}\underset{OH}{CH}\text{-}CH_2\text{-}Y\right]_n\!R^2 \quad (I),$$

dans laquelle n désigne un nombre de 1 à 100, Y représente un radical -N($R^3$)-, -S- ou -S-$R^4$S-, $R^1$ représente un alkylène en $C_2$-$C_8$ qui peut éventuellement être interrompu par 1 à 3 atomes d'oxygène de fonction éther, $R^2$ représente l'hydrogène ou un alkyle en $C_1$-$C_8$ qui ne porte pas de substituant ou qui porte de 1 à 5 radicaux hydroxy et qui peut éventuellement être interrompu une ou plusieurs fois par -O-, -S- ou -N($R^5$)-, avec la condition que lorsque Y représente -S- et que n est égal à 1 le symbole $R^2$ ne puisse pas représenter un alkyle en $C_1$-$C_8$ non substitué, $R^3$ représente l'hydrogène ou un alkyle en $C_1$-$C_8$ qui ne porte pas de substituant ou qui porte de 1 à 5 radicaux hydroxy et qui peut éventuellement être interrompu une ou plusieurs fois par -O-, -N($R^5$)- ou -S-, $R^4$ représente un alkylène en $C_2$-$C_8$ qui ne porte pas de substituant ou qui peut porter un ou plusieurs radicaux -OH et qui peut éventuellement être interrompu, une ou plusieurs fois, par -O- ou -S-, et $R^5$ a la même signification que $R^3$ mais sans pouvoir représenter un alkyle interrompu par des hétéroatomes.

2. Composés selon la revendication 1, dans lesquels l'indice n, dans la formule I, est supérieur à 1.

3. Composés selon la revendication 1, dans lesquels Y, dans la formule I, représente un radical -N($R^3$)-.

4. Composés selon la revendication 1, dans lesquels le symbole $R^3$, dans la formule I, représente un alkyle en $C_1$-$C_8$ qui porte de 1 à 5 radicaux hydroxy

et qui est éventuellement interrompu par -N($R^5$)- une ou plusieurs fois.

5. Composés selon la revendication 4, dans lesquels le symbole $R^3$, dans la formule I, représente un alkyle qui contient 6 atomes de carbone et qui porte 5 radicaux hydroxy.

6. Composés selon la revendication 1, caractérisés en ce que, dans la formule I, Y représente -N($R^3$)- ou -S-, $R^1$ représente un éthylène, $R^2$ représente l'hydrogène ou un alkyle en $C_1$-$C_6$ qui ne porte pas de substituants ou qui porte de 1 à 5 radicaux hydroxy et qui est éventuellement interrompu par 1 à 3 radicaux -O- ou -S-, et $R^3$ représente l'hydrogène ou un alkyle en $C_1$-$C_8$ porteur de 1 à 5 radicaux hydroxy, avec la condition que $R^2$ ne puisse pas être un alkyle en $C_1$-$C_6$ non substitué lorsque Y représente -S- et que n est égal à 1.

7. Composés selon l'une quelconque des revendications 1 à 5, caractérisés en ce que, dans la formule I, n a une valeur telle que la viscosité cinématique d'une solution à 50% dans de l'eau, à 40°C, soit comprise entre 20 et 50 $mm^2/s$.

8. Composés selon l'une quelconque des revendications 1 à 5, caractérisés en ce que, dans la formule I, n a une valeur telle que la viscosité cinématique d'une solution à 50% dans de l'eau, à 40°C, soit comprise entre 25 et 35 $mm^2/s$.

9. Composés selon l'une quelconque des revendications 1 à 5, caractérisés en ce que, dans la formule I, n a une valeur telle que la viscosité cinématique d'une solution à 50% dans de l'eau, à 40°C, soit comprise entre 27 et 30 $mm^2/s$.

10. Application des composés de formule I selon la revendication 1, comme additifs hautes pressions et antiusures dans des liquides fonctionnels polaires.

11. Composition qui contient un liquide fonctionnel polaire et au moins un composé de formule I selon la revendication 1.

12. Composition selon la revendication 11, dans laquelle le liquide fonctionnel polaire est à base d'eau, d'éthylène-glycol, de diéthylène-glycol, de polyéthylèneglycols et de leurs mélanges avec de l'eau, ou à base d'esters phosphoriques, ou est un liquide fonctionnel de structure complexe.

13. Composition selon la revendication 12, caractérisée en ce que, dans le liquide fonctionnel de structure complexe, la teneur en eau est d'au moins 50% en poids et en ce qu'elle contient, comme autres composantes, de l'acide phosphorique et une base servant à ajuster la valeur du pH entre 7,5 et 12.

14. Composition selon la revendication 13, dans laquelle le liquide fonctionnel de structure complexe contient en outre de l'acide oléique et de l'acide borique.

15. Composition selon la revendication 11, dans laquelle la quantité des composés de formule I est comprise entre 0,5 et 10% en poids.

16. Produits réactionnels qui peuvent être obtenus par réaction de bis-thio-éthers glycidyliques de formule:

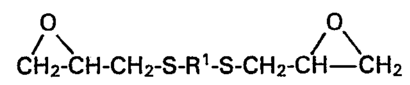

avec des composés de formule

$$H\text{-}Y\text{-}R^2,$$

formules dans lesquelles Y représente -N($R^3$)-, -S- ou -S-$R^4$-S-, $R^1$ représente un radical alkylène en $C_2$-$C_8$ qui peut éventuellement être interrompu par 1 à 3 atomes d'oxygène de fonction éther, $R^2$ représente l'hydrogène ou un alkyle en $C_1$-$C_8$ qui ne porte pas de substituant ou qui porte de 1 à 5 radicaux hydroxy et qui peut éventuellement être interrompu une ou plusieurs fois par -O-, -S- ou -N($R^5$)-, $R^3$ représente l'hydrogène ou un alkyle en $C_1$-$C_8$ qui ne porte pas de substituiant ou qui porte de 1 à 5 radicaux hydroxy et qui peut éventuellement être interrompu une ou plusieurs fois par -O-, -N($R^5$)- ou -S-, $R^4$ représente un alkylène en $C_2$-$C_8$ qui ne porte pas de substituant ou qui peut porter un ou plusieurs radicaux -OH et qui peut éventuellement être interrompu, une ou plusieurs fois, par -O- ou -S-, et $R^5$ a la même signification que $R^3$ mais sans pouvoir représenter un alkyle interrompu par des hétéroatomes, la réaction étant effectuée de telle façon que, lorsque Y représente S et que $R^2$ représente un alkyle en $C_1$-$C_8$ non substitué, il y ait, dans le produit réactionnel, au moins 2 motifs de bis-thio-éthers glycidyliques par molécule.